# EUROPEAN PATENT APPLICATION

(11) **EP 3 427 662 A1**
(43) Date of publication of application: **16.01.2019**
(21) Application number: 17763369.0
(22) Date of filing: 09.03.2017
(51) Int. Cl.: A61B 5/15, A61B 5/0225, A61M 5/00

(54) **SIMPLE AUTOMATIC ELECTRONIC TOURNIQUET**

(30) Priority: 10.03.2016 JP 2016047318
(71) Applicant: Masaki Nobuyuki, Chiba-shi, Chiba 267-0066 (JP)
(72) Inventor: Masaki Nobuyuki, Chiba-shi, Chiba 267-0066 (JP)
(74) Representative: SSM Sandmair
(86) International application number: PCT/JP2017/009452
(87) International publication number: WO 2017/155029

(57) **Abstract**

A simple auto electronic tourniquet is to be provided, which, during the preparation of venipuncture, does not give pain to persons to which avascularization is performed, shorten the preparation time of venipuncture, and appropriately perform avascularization. The simple auto electronic tourniquet of the present invention comprises a cuff, a pressurizing means for gradually raising the internal pressure of the cuff, a depressurizing means for gradually decreasing the internal pressure of the cuff, a pressure detecting means for detecting the internal pressure of the cuff, a target pressure setting means for setting the internal pressure of the cuff to a target pressure, a pressure control means for controlling the internal pressure of the cuff so that it gets close to the approximate value of the target pressure, and a notifying means for notifying that venipuncture is performable.

In order to avoid excessive pressures, long-time performance of avascularization, sudden rise of pressure, which may cause patients to feel stressed, the width of the cuff of the tourniquet of the present invention shall be 75mm or less, and more preferably in the range between 10mm and 60mm.

## Description

### [FIELD OF THE INVENTION]

The present invention relates to a simple auto electronic tourniquet which appropriately and easily performs venipuncture, blood collection, or securing of vein root (securing of blood vessel).

Conventionally, avascularization is performed by placing an elastic band around the limb in such as a blood collection site. The elastic band is tightened with a degree of force, however, in the current situation, what degree of tightening force is required has to be decided with depending on experience and intuition of nurse. Therefore, the tightening force is unstable, either too weak or too strong, and it has been difficult to tighten the limb with a moderate avascularization pressure.

A tourniquet has been known having a means for assisting tightening the limb with a moderate avascularization pressure, in which a rubber tube or rubber belt is provided with some lines drawn in one or two colors in appropriate intervals repeatedly as an index (Patent Document 1).

On the other hand, another tourniquet that is automatically able to operate to some extent has been known, which comprises a manchette mountable to the limb, a pressure-applying means for pressurizing the manchette, a blood pressure-measuring means for measuring systolic blood pressure (maximal blood pressure) and diastolic blood pressure (minimal blood pressure) by pressurizing and/or depressurizing the limb with the pressure-applying means, where diastolic blood pressure and systolic blood pressure is measured by the blood pressure measuring means, and avascularization is performed by setting up the pressure of the limb between the systolic blood pressure (Patent Document 2)

In the tourniquet constructed in the above-mentioned way, a manchette is mounted on the limb; the manchette is pressurized to a pressure well beyond the expected value of systolic blood pressure; the manchette is gradually depressurized; and during the depressurizing process, values of systolic blood pressure and diastolic pressure are measured again based on the change of a biological signal.

Namely, during the depressurizing process, as the change of a biological signal such as the generation of Korotkov sounds can be detected, a value of systolic blood pressure is detected in the pressure of the c manchette at the time of detecting the generation of Korotkov sounds. By continuing to depressurize the manchette, then as the disappearance of Korotkov sounds can be detected, a value of diastolic blood pressure is detected in the pressure of the manchette at the time of detecting the disappearance of Korotkov sounds. Then, by pressurizing the manchette again, avascularization is performed by setting the pressure of the manchette in a predetermined pressure between the values of diastolic blood pressure and systolic blood pressure.

By performing avascularization as described above, since the blood flow in the vein can be stopped while the blood flow in the artery being maintained, the vein can be surely expanded and easily punctured with an injection needle.

In using the above-mentioned tourniquet, at the time of detecting a value of systolic blood pressure of a patient, a pressure high enough to exceed "said value" of systolic blood pressure has to be applied to the manchette, and therefore the patient may feel pain in his/her limb, which is a problem. Besides, as firstly the patient's both values of systolic blood pressure and diastolic blood pressure are measured, and then the pressure of the manchette is set to a predetermined pressure between them. Therefore, a long time may be required to prepare venipuncture.

The present applicant has previously proposed an auto tourniquet detecting a change of a biological signal relating to diastolic blood pressure, which is configured such that a pressure setting and maintaining circuit sets the pressurizing force of a surrounding member measured at the time of detecting a change of the biological signal by a diastolic blood pressure detecting circuit as a target pressurizing force and also controls the surrounding member so that its pressurizing force falls within the range similar to the target pressuring force (Patent Document 3). The configuration does not require measuring both of the systolic blood pressure and diastolic blood pressure of a patient, shortens the preparation time for venipuncture, and decreases burden of the patient.

However, the configuration requires further improvements. Namely, as the configuration is to maintain an appropriate value of avascularization pressure by detecting biological signals continuously, it become not possible either to detect the biological signals or to perform avascularization appropriately due to some kind of troubles such as outside vibrations or the patient's sudden position changes.

### [PRIOR ART DOCUMENTS]

### [PATENT DOCUMENTS]

[PATENT DOCUMENT 1] Publication of Registered Utility Model Application No. 3150237 (JP)
[PATENT DOCUMENT 2] Patent Application Publication 2009-95516 (JP)
[PATENT DOCUMENT 3] Patent Application Publication 2013-118938 (JP)

### [SUMMARY OF THE INVENTION]

### [PROBLEM TO BE SOLVED BY THE INVENTION]

The present invention is made in view of the above-described circumstances, and its object is to provide a simple auto electronic tourniquet which, during the preparation of venipuncture, does not give pain to persons for which avascularization is performed, shortens the preparation time of venipuncture, and appropriately performs avascularization.

In order to solve the above-mentioned problems, according to a first aspect of the present invention, an auto electronic tourniquet comprises a cuff, a pressurizing means to gradually raise the internal pressure of the cuff, a depressurizing means to gradually decrease the internal pressure of the cuff, a pressure detecting means to detect the internal pressure of the cuff, a target pressure setting means to set the internal pressure of the cuff to a target pressure, a pressure control means to control the internal pressure of the cuff so that it gets close to the approximate value of the target pressure, and a notifying means to notify that venipuncture is performable.

According to a second aspect of the present invention, in the first aspect thereof, the depressurizing means is able to depressurize the internal pressure of the cuff by operating an operating device provided off the cuff.

According to a third aspect of the present invention, in either the first or second aspect thereof, the width of the cuff is 75mm or less.

According to a fourth aspect of the present invention, in either the first or second aspect thereof, the capacity of the cuff is 80cc or less.

According to a fifth aspect of the present invention, in either one among the first to fourth aspects thereof, the target pressure setting means is able to select an arbitrary fixed value by operating from the outside.

According to a sixth aspect of the present invention, in either one among the first to fourth aspects thereof, a biological signal detection means is further provided, and the target pressure setting means sets the target pressure based on the result of detection of biological signals by operation of the biological signal detection means.

According to a seventh aspect of the present invention, in either one among the first to fourth aspects thereof, the biological signal detection means is further provided; the target pressure setting means is able to select an arbitrary fixed value by operating from the outside; the biological signal detection means detect the biological signal in a condition pressurized with a value of the internal pressure of the cuff corresponding to the arbitrary fixed value; and the notifying means notify that venipuncture is performable when the biological signal detection means detects the biological signal.

According to an eighth aspect of the present invention, in either one among the first to fourth aspects thereof, a biological signal detection means is further provided; the target pressure setting means is able to select an arbitrary fixed value by operating from the outside; the biological signal detection means detect a biological signal in a condition pressurized with a value of the internal pressure of the cuff corresponding to the arbitrary fixed value; and when the biological signal is not detected, the target pressure setting means resets the internal pressure of the cuff to a target pressure which is higher than the previously selected arbitrary fixed value.

### [EFFECT OF THE INVENTION]

According to the first aspect of the present invention, an appropriate avascularization pressure may be realized without depending on experience and intuition of nurse. Besides, as the internal pressure of the cuff gradually increases, patients can avoid sudden and strong tightening of the cuff, by which patient's mental and physical stress may be relieved. In particular, patients having delicate skin can be relieved from excessive stimulus to the skin, and hairy patients can be relieved from the pain which they sense when their body hair is pulled.

According to the second aspect of the present invention, when venipuncture is completed and the internal pressure of the cuff is released, as the internal pressure of the cuff can be depressurized without touching the cuff, such an accident may be prevented that the needle kept in the blood vessel slips out thereof. Also, operability of the tourniquet for nurse may improve.

According to the third aspect of the present invention, while the amount of material for the cuff is reduced, a degree of pressurized state from which patients feel less stressed may be realized. In particular, since avascularization is performed not for the purpose of sphygmomanometry but for the purpose of venipuncture, it is not necessary to adopt a cuff having a predetermined width for synchronizing the internal pressure of the cuff to the internal pressure of the blood vessel.

According to the fourth aspect of the present invention, while the amount of material for the cuff is reduced, a degree of pressurized state from which patients feel less stressed may be realized. In particular, avascularization is performed not for the purpose of sphygmomanometry but for the purpose of venipuncture, it is not necessary to adopt a cuff having a volume to realize a predetermined width for synchronizing the internal pressure of the cuff to the internal pressure of the blood vessel.

According to the fifth aspect of the present invention, as the target value of the internal pressure of the cuff can be set by operating from the outside, it is expected that the performance of avascularization with depending on experience and intuition of nurse may be avoided and that avascularization with an appropriate pressure may be performed.

According to the sixth aspect of the present invention, as the target value of the internal pressure of the cuff is set based on such changes in biological signals detected by the tourniquet as the change of internal pressure vibration of the cuff (=oscillation) or the generation or degree of Korotkov sounds, it is expected that the performance of avascularization with depending on experience and intuition of nurse may be avoided and that avascularization with an appropriate pressure may be performed.

According to the seventh aspect of the present invention, although blood pressure differs from person to person, the appropriate internal pressure of the cuff for many of the adult patients falls within an empirical range. Therefore, by simply setting the target pressure which takes the steps of setting the target value of the internal pressure of the cuff to a fixed value, conducing detections of the generation of states of biological signals which could be generated in the event that avascularization is suitably performed, and notifying that punctuation is performable in the event the generation of states of biological signals is detected, desired conditions of avascularization may be surely notified.

Furthermore, according to the eighth aspect of the present invention, if desired conditions of avascularization are not obtained by setting the internal pressure of the cuff to a fixed value, by additionally raising internal pressure of the cuff, appropriate conditions of avascularization may be obtained.

In the present invention, "gradual pressurizing" means gradually raising the internal pressure of the cuff, whether the pressure rising speed is high or low. For example, it means raising the pressure of the cuff with a tourniquet which is different from either a rubber tube or belt-formed tourniquet in configuration so that it prevents the limb of patients from suddenly tightened. The pressurizing methods include but not limited to using a compressed air. "Gradual depressurizing" means gradually reducing the internal pressure of the cuff, whether the pressure reducing speed is high or low.

A specific configuration to implement the "target pressure setting means" includes an operational button provided off the cuff. Although it is operationally convenient for a target pressure to be pre-set in each operating device, it may comprise a control panel for inputting a particular target pressure with a numerical keypad or a touch panel.

"Pressure control means" refers to means to generally match the internal pressure of the cuff measured by a pressure sensor detecting thereof and the target pressure set by the above-mentioned target pressure setting means by comparing both of them.

"Notifying means" may include but not limited to an audible notifying means such as a buzzer and/or a voice, and lighting or flashing of the LED lamp, stimulation to other human sensor, and the combined use of them.

"Width of the cuff" refers to the area in a direction parallel with the limb when the tourniquet of the present invention is mounted thereon. As the area is broader, the length of the blood vessel in the limb to be constricted becomes longer, and a desired performance of avascularization can be realized by a relatively low pressure from the external, however, the internal pressure of the cuff is raised by air pressure, larger amount of air must be supplied into the cuff. On the contrary, when the area is smaller, smaller amount of air can raise the internal pressure of the cuff, however, as the length of the blood vessel in the limb to be constricted becomes shorter, a desired performance of avascularization needs a relatively high pressure from the external. Namely, in order to avoid excessive pressures, long-time performance of avascularization, sudden rise of pressure, which may cause the patients to feel stressed, the width of the cuff of the tourniquet of the present invention is 75mm or less. More preferably, it may be in the range between 10mm and 60mm. If the width is less than 10mm, as the internal pressure of the cuff relatively becomes excessively high and the capacity of the cuff also become smaller, rising rate of the internal pressure of the cuff becomes too high, causing the patients to feel more stressed. Further, as the higher internal pressure of the cuff is required, the cuff itself receives a larger load and may be difficult to obtain desired durability. On the other hand, if the width is longer than 75mm, the cuff may lose portability in carrying or mounting and may also require longer time in increasing its internal pressure, causing the patients to feel more stressed.

"Arbitrary fixed value" may be set in multiple stages to, for example, 100mmHg, 150mmHg, and 200mmHg. It is noted that although those fixed values are higher than the systolic blood pressure of adult in some cases, it is because the tourniquet of the present invention is not originally intended for the purpose of conducting sphygmomanometry. In conducting sphygmomanometry, the cuff is required to have a specific width, however, the present invention is intended for the purpose of performing avascularization appropriately.

Although "biological signals" to be detected are intended to be pulsations (oscillation), Korotkov sounds may be included. Pulsations (oscillations) with strengths above a certain level may be set to a subject of the detection of "a predetermined biological signal". Alternatively, while pulse waves are detected over time after the start of pressurization of the cuff, and with maximum pulse waves being detected over time, "a predetermined biological signal" may be detected. Further, when a pulse wave, which is detected at a predetermined time, is stronger than the by-then-detected maximum pulse wave at a given rate or higher, "a predetermined biological signals" may be detected.

### [BRIEF DESCRIPTION OF DRAWINGS]

[FIG. 1] a view showing an auto electronic tourniquet of the present invention in the mounting condition
[FIG. 2] a functional block diagram of an auto electronic tourniquet of the present invention
[FIG. 3] an external view of an auto electronic tourniquet of the present invention
[FIG. 4] an external view of the cabinet of an auto electronic tourniquet of the present invention
[FIG. 5] a first flow chart showing a series of operations from the start of pressurization, notification of availability of venipuncture, to the gradual depressurization
[FIG. 6] a second flow chart showing a series of operations from the start of pressurization, notification of availability of venipuncture, to the gradual depressurization
[FIG. 7] a third flow chart showing a series of operations from the start of pressurization, notification of availability of venipuncture, to the gradual depressurization
[FIG. 8] a conception diagram showing a relationship between width of the cuff and the necessary internal pressure of the cuff in a tourniquet

### [MODE FOR CARRYING OUT THE INVETNION]

### [EXAMPLE 1]

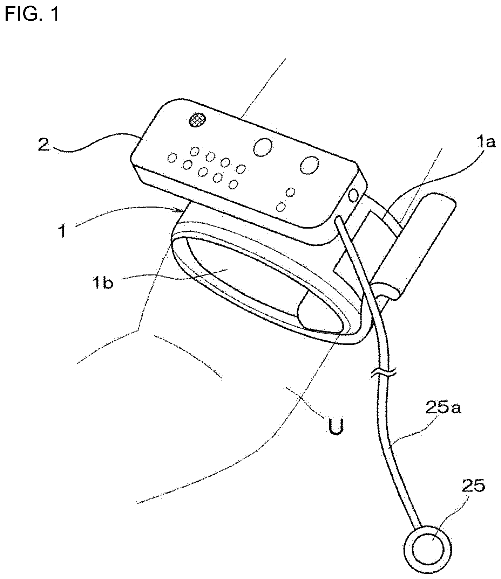

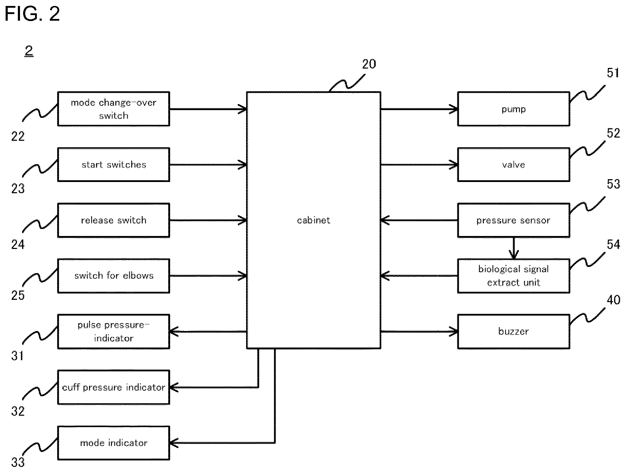

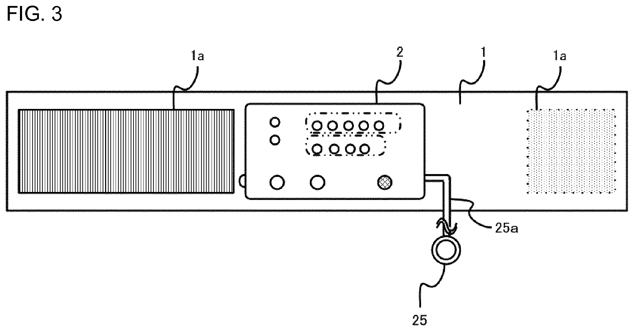

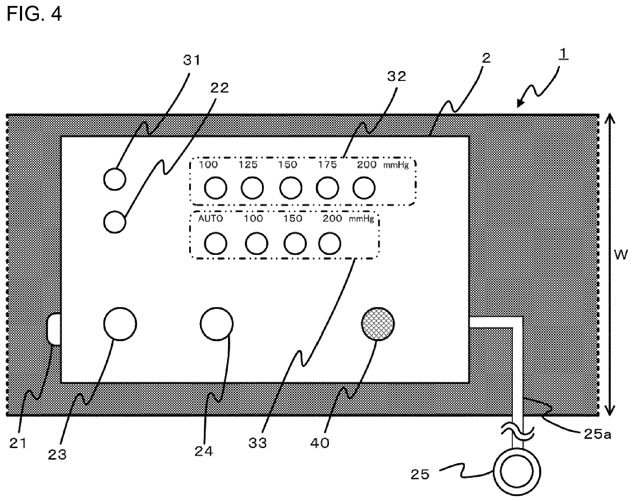

An auto electronic tourniquet shown as the first embodiment of the present invention, as described in FIGs1-4, has a manchette (cuff) 1 mountable around an upper arm U which is one of the limbs of the human body and a cabinet 20 integrally provided with the cuff 1. The cabinet 20 has a controller 2 inside.

The cuff 1 is configured to be maintained in a pipe-like shape by a hook-and-loop fastener 1a when mounted around the upper arm U and to be easily detachable to and/or from the upper arm U. Also, the cuff 1 is provided with a pressure bag 1b at a portion thereof which is to be the inner surface when formed in a pipe-like shape. The pressure bag 1b provides pressurizing force to the upper arm U. The pressure bag 1b is provided with a pump 51 for supplying air pressure to the pressure bag 1b and a valve 52 for adjusting pressure in the pressure bag 1b. The pressure bag 1b is further provided with a pressure sensor 53 for detecting the inner pressure of the cuff and a biological signal extraction unit 54 for extracting and detecting only a portion of the pressure detected by the pressure sensor 53 which corresponds to a pulse pressure.

The cabinet 20 is provided with the controller 2 having hardware resources of a computer. The controller executes software with sequence program, thereby various controls described hereinafter are performed.

A power circuit (not shown in the drawings) is started by pushing down a power switch 21. A mode change-over switch 22 is for the purpose of switching binary mode, i.e., "auto mode" and "manual mode". The "manual mode" is further set to "100mmHg", "150mmHg", and "200mmHg" as the fixed values for the target internal pressure of the cuff. Whenever the mode change-over switch is pushed down, change of the mode is possible. A pump 51 is started by pushing down a start switch 23, and air discharged from the pump 51 is filled into the cuff. When an operation of pressure release is needed in the cabinet 20, the valve 52 is thrown open by pushing down a release switch 24 and the internal pressure of the cuff decreases.

A switch for elbows 25 is connected to the cabinet 20 through a lead wire 25a and has a common function with the start switch 23 and the release switch 24. Namely, by pushing down the switch for elbows 25 once, pressurization of the cuff is started. Also, by pushing down the switch for elbows 25 again after the completion of venipuncture, the valve 52 is opened and the cuff is depressurized. When venipuncture is performed, as injectors, syringes and/or gauzes will usually occupy both hands of nurses and/or medical doctors, and an operating means for the internal pressurization/depressurization of the cuff which is operatable other than by hands has been desired. By using the switch for elbows 25, the internal pressure of the cuff may be released safely and surely while venipuncture is performed. The cuff 1 and the cabinet 20A constitute a cuff section, and the switch for elbows 25 corresponds to an operating device provided on other than the cuff section.

A pulse pressure indicator 31 is an LED lamp and flashes on and off in response to the presence or absence of biological signals (pulse pressures) extracted by the biological signal extraction unit 54 from the pressures detected by the pressure sensor 53. Thereby, whether avascularization is appropriately performed is visible. Further, the pulse pressure indicator 31 may be configured to be lighted at the time when avascularization is finished, namely when venipuncture becomes possible. In this case, the pulse pressure indicator 31 functions as a notifying means. Alternatively, when unusual situation occurs such as excessive pressurization, insufficient pressure, or a lack of battery, the pulse pressure indicator 31 may be functionalized as a means to notify the unusual situation by high-speed flashing. However, those additional notifying functions are not necessarily used jointly with the pulse pressure indicator 31 and may be performed by using a cuff pressure indicator 32 or a mode indicator 33 (both of which are described hereinafter)or by jointly using both or either of them.

The cuff pressure indicator 32 consists of a plurality of LED lamps and the LED lamps are lighted in order in response to the internal pressure of the cuff detected by the pressure sensor 53. Also, the mode indicator 33 consists of a plurality of LED lamps, and one of the LED lamps corresponding to the current mode [either automatic (automatic operation), 100mmHg (manual operation), 150mmHg (manual operation), or 200mmHg (manual operation)] is lighted and notifies the current mode in response to the operation of the mode change-over switch 22.

When avascularization is finished and venipuncture is possible, a buzzer 40 rumbles and notifies the user the situation.

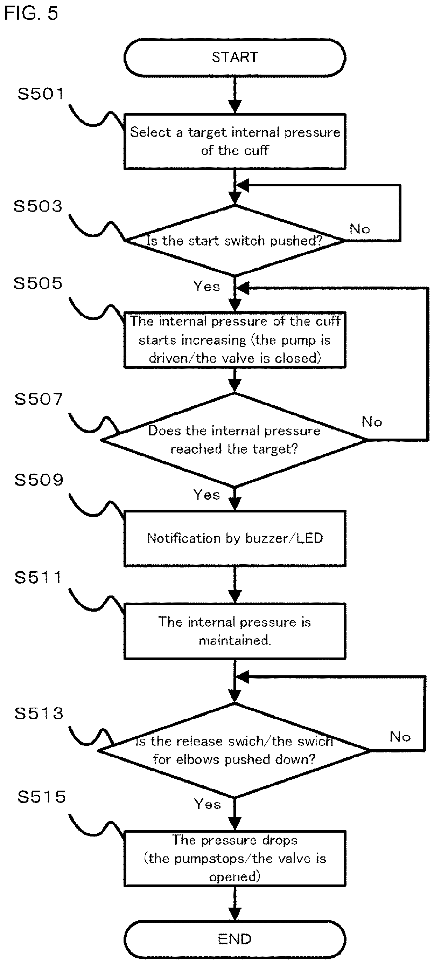

Hereinafter, operation of an auto electronic tourniquet with using a fixed value of avascularization pressure selected by manual operation will be explained with reference to Fig. 5.

A target internal pressure of the cuff is selected from the plurality of fixed values set in the manual mode by pushing down the mode change-over switch 22 (step S501). The mode change-over switch 22 functions as a target pressure setting means. Then, when the start switch 23 is pushed down (step S503: Yes), the pump 51 is driven and the valve 52 is completely "closed" and the internal pressure of the cuff starts increasing (step S505). While increasing, the internal pressure of the cuff is detected by the pressure sensor 53, and when it reaches a setting pressure (step S507: Yes), the completion of avascularization is notified (step S509). The notifying means may be to light either of the above-described indicators or may be to rumble the buzzer 40. Also, the operation of the pump 51 stops and the valve 52 remained "closed" to maintain the internal pressure of the cuff (step S511). Not shown in the Figures, although the internal pressure of the cuff is maintained in a pressure approximate to the target internal pressure of the cuff by closing the valve 52, it may be configured such that the pressure sensor 53 continues to detect the internal pressure of the cuff in preparation for slight leak of air, and when the difference between the internal pressure of the cuff and the target internal pressure reaches a predetermined value, the pump 51 is driven again. The pump 51 and valve 52 collaborate with the controller 2 and functions as a pressurizing means and a pressure control means.

After venipuncture is performed in this state, the release of the internal pressure of the cuff is usually needed in a state that the upper arm U is still punctured. Nurses and/or medical doctors who perform venipuncture release the pressure by pushing down either the release switch 24 or the switch for elbows 25. When either the release switch 24 or the switch for elbows 25 is pushed down (step S513: Yes), the valve is "opened" and the internal pressure of the cuff drops (step S515). The release switch 24, switch for elbows 25, and valve 52 collaborate with the controller 2 and function as a depressurizing means.

### [EXAMPLE 2]

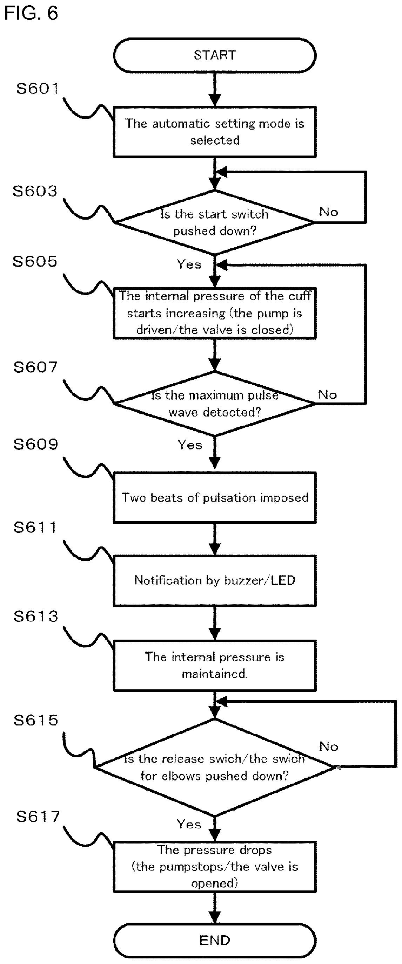

Then, an operation of the auto electronic tourniquet with automatically setting a target avascularization pressure based on biological signals will be explained with reference to Fig. 6.

The automatic setting mode is selected by pushing down the mode change-over switch 22 (step S601). Then, when the start switch 23 is pushed down (step S603: Yes), the pump 51 is driven and the valve 52 is completely "closed", and the internal pressure of the cuff starts increasing (step S605).

The pressure sensor 53 detects the internal pressure of the cuff during its increase, and the biological signal extraction unit 54 extracts and detects only a portion of the pressure values detected by the pressure sensor 53 which corresponds to a pulse wave. Deflection of the detected pulse wave is recorded over time, and when the maximum pulse wave is detected as a result of continuous detection (step S607: Yes), and with a time interval (buffer) corresponding to two beats of pulsation being imposed (step S609), the completion of avascularization is notified (step S611). The time interval (buffer) is not limited to using beat rate and may be configured to delay the operation by a predetermined second (i.e. one second). In this embodiment, the pressure sensor 53 and the biological signal extraction unit 54 collaborate with the controller 2 and functions as a target pressure setting means. The notifying means may be to light either of the above-described indicators or may be to rumble the buzzer 40. Also, the operation of the pump 51 stops and the valve 52 remained "closed" to maintain the internal pressure of the cuff (step S613). As in the first embodiment, in order to maintain the internal pressure of the cuff, a configuration is adopted such that the internal pressure of the cuff is continuously monitored by the pressure sensor 53, and when it decreases by a predetermined range or more, the pump 51 is driven to additionally pressurize it.

After venipuncture is performed in this state, the release of the internal pressure of the cuff is usually needed in a state that the upper arm U is still punctured. Nurses and/or medical doctors who perform venipuncture release the pressure by pushing down either the release switch 24 or the switch for elbows 25. When either the release switch 24 or the switch for elbows 25 is pushed down (step S615: Yes), the valve is "opened" and the internal pressure of the cuff drops (step S617).

### [EXAMPLE 3]

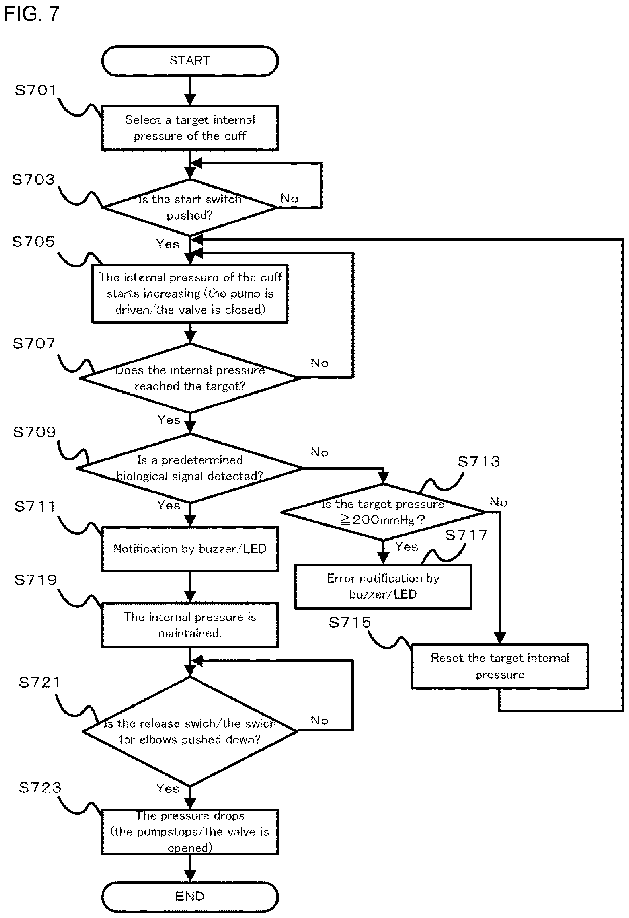

Then, hereinafter, another operation of an auto electronic tourniquet of the present invention will be explained with reference to Fig. 7, in which while a fixed value of avascularization pressure is selected by manual operation, whether avascularization is performed in an appropriate condition is detected by considering biological signals.

A target internal pressure of the cuff is selected from the plurality of fixed values set in the manual mode by pushing down the mode change-over switch 22 (step S701). Then, when the start switch 23 is pushed down (step S703: Yes), the pump 51 is driven and the valve 52 is completely "closed" and the internal pressure of the cuff starts increasing (step S705). While increasing, the internal pressure of the cuff is detected by the pressure sensor 53. When it reaches the setting pressure in the manual mode (step S707: Yes) and a predetermined biological signal is detected by the pressure sensor 53 and the biological signal extract unit 54 (step S709: Yes), the completion of avascularization is notified (step S711). The notifying means may be to light either of the above-described indicators or may be to rumble the buzzer 40. If a pulse wave with strength in a predetermined level or more is detected at the time when the internal pressure of the cuff reaches the setting pressure in the manual mode, "a predetermined biological signals" may be assumed to be detected. Alternatively, it may be configured such that pulse waves are detected over time by the biological signal extraction unit 54, and if a maximum pulse wave is detected during the detection, a predetermined biological signals" may be assumed to be detected. Further, it may be configured such that pulse waves are detected over time by the biological signal extraction unit 54, and if a maximum pulse wave is detected during the detection and if a pulse wave at the time when the internal pressure of the cuff reaches the setting pressure in the manual mode falls within a predetermined range of maximum pulse wave, "a predetermined biological signal" may be assumed to be detected.

When a predetermined biological signal is not detected by the pressure sensor 53 and the biological signal extract unit 54 (step S709: No), resetting the value of the target internal pressure of the cuff is conducted (step S715). A resetting value of the target internal pressure of the cuff shall be a value which adds 50mmHg to the original setting pressure in the manual mode. However, if the value of the setting pressure in this stage has been set to "200mmHg" (step S713: Yes), the increase of pressure is not to be conducted, and an error is notified (step S717). When a predetermined biological signal is detected, the operation of the pump 51 stops and the valve 52 remained "closed" to maintain the internal pressure of the cuff (step S719).

After venipuncture is performed in this state, the release of the internal pressure of the cuff is usually needed in a state that the upper arm U is still punctured. Nurses and/or medical doctors who perform venipuncture release the pressure by pushing down either the release switch 24 or the switch for elbows 25. When either the release switch 24 or the switch for elbows 25 is pushed down (step S721: Yes), the valve is "opened" and the internal pressure of the cuff drops (step S723).

In the auto electronic tourniquet of the present invention, its characteristic feature is that the width of the cuff is configured to be 75mm or less. The technical significance of this point will be mentioned below.

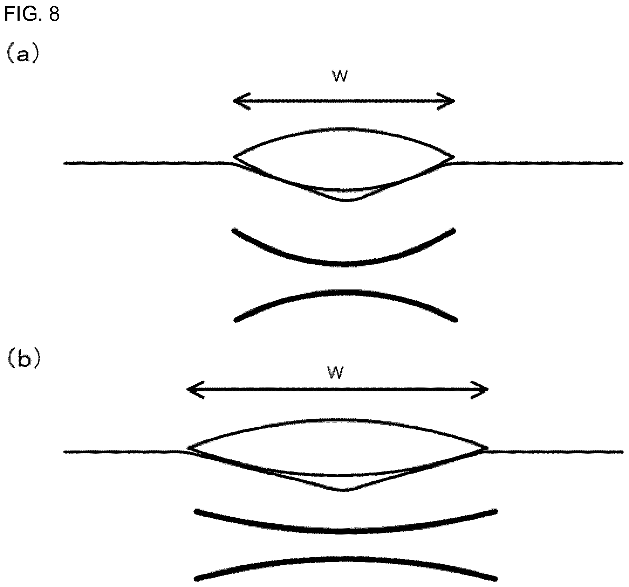

The width W of the cuff of a tourniquet is preferably smaller from the viewpoint of easy mounting and short pressurizing time (FIG. 8 (a)). This point may be an advantage for the auto electronic tourniquet of the present invention which is not placed under the restrictions under which the common sphygmomanometers are placed (refer to FIG. 8 (b)). Namely, when a cuff with smaller width is used in conducting sphygmomanometry, measured values of blood pressure have to be higher. This is conceived that if pressurized area is smaller, higher pressure is required for providing a stenosis to blood vessel (a physical transformation of blood vessel) to the same extent as pressurized area being not smaller. In this regard, the object of the auto electronic tourniquet of the present invention is to provide a stenosis to blood vessel in relation to the internal pressure of blood vessel, and not to measure mean blood pressure. Further, changes of pulsation amplitude of internal pressure of the cuff is produced by a vascular stenosis of the pressurized portion of blood vessel, and when pulsation amplitude becomes maximum, an external force similar to the mean value of the internal pressure of blood vessel is applied. Namely, if a pressurized state of detecting a maximum value of pulse wave is realized, an optimum state for avascularization will be realized. In order not to make patients feel excessively stressed by the optimum avascularization state, while having studies the time until the internal pressure of the cuff reaches its maximum value and/or its target value, we have found that the width of the cuff equal to or less than 75mm may produce a desired avascularization state in a short time without making patients feel unnecessary stress. Still, when the width W of the cuff is too small, as a strong tightening force is applied to a small area of the limb of a patient, the patient feeling more stressed. In addition, as a high internal pressure of the cuff is required, durability of the pressurized bag may be spoiled. Thus, the width of the cuff is preferably 10mm or more, and it is also preferably 60mm or less for prompt realization of an optimum avascularization state.

### [DESCRIPTION OF THE REFERENCE NUMERALS]

- 1: manchette (cuff)
- 2: controller
- 20: cabinet
- 21: power switch
- 22: mode change-over switch
- 23: start switches
- 24: release switch
- 25: switch for elbows
- 25a: lead
- 31: pulse pressure-indicator
- 32: cuff pressure indicator
- 33: mode indicator
- 40: buzzer
- 51: pump
- 52: valve
- 53: pressure sensor
- 54: biological signal extract unit

## Claims

1. A simple auto electronic tourniquet comprises:
a cuff,
a pressurizing means for gradually raising the internal pressure of a cuff,
a depressurizing means to gradually decreasing the internal pressure of the cuff,
a pressure detecting means for detecting the internal pressure of the cuff,
a target pressure setting means for setting the internal pressure of the cuff to a target pressure, and
a pressure control means for controlling the internal pressure of the cuff so that it gets close to the approximate value of the target pressure, and
a notifying means for notifying that venipuncture is performable.

2. The auto electron tourniquet as claimed in Claim 1, wherein the depressurizing means is able to depressurize the internal pressure of the cuff by operating an operating device provided off the cuff.

3. The simple auto electron tourniquet as claimed in Claim 1 or 2, wherein the width of the cuff is 75mm or less.

4. The simple auto electron tourniquet as claimed in either Claim 1 or 2, wherein the capacity of the cuff is 80cc or less.

5. The simple auto electron tourniquet as claimed in either one among Claims 1 to 4, wherein the target pressure setting means is able to select an arbitrary fixed value by operating from the outside.

6. The simple auto electron tourniquet as claimed in either one among Claims 1 to 4, wherein a biological signal detection means is further provided, and the target pressure setting means sets the target pressure based on the result of detection of biological signals by operation of the biological signal detection means.

7. The simple auto electron tourniquet as claimed in either one among Claims 1 to 4, wherein a biological signal detection means is further provided; the target pressure setting means is able to select an arbitrary fixed value by operating from the outside; the biological signal detection means detect a biological signal in a condition pressurized with a value of the internal pressure of the cuff corresponding to the arbitrary fixed value; and the notifying means notify that venipuncture is performable when the biological signal detection means detects the biological signal.

8. The simple auto electron tourniquet as claimed in either one among Claims 1 to 4, wherein a biological signal detection means is further provided; the target pressure setting means is able to select an arbitrary fixed value by operating from the outside; the biological signal detection means detect a biological signal in a condition pressurized with a value of the internal pressure of the cuff corresponding to the arbitrary fixed value; and when the biological signal is not detected, the target pressure setting means resets the internal pressure of the cuff to a target pressure which is higher than the previously selected arbitrary fixed value.
